# EUROPÄISCHE PATENTSCHRIFT

(11) **EP 0 557 831 B1**
(45) Veröffentlichungstag und Bekanntmachung des Hinweises auf die Patenterteilung: **22.01.1997**
(21) Anmeldenummer: 93102269.3
(22) Anmeldetag: 12.02.1993
(51) Int. Cl.: G06F 17/00, G06F 159/00

(54) **Gerät zur Bestimmung des optimalen Entbindungstermins**
Apparatus for determination of the optimal delivery date
Appareil pour déterminer la date d'accouchement optimale

(30) Priorität: 25.02.1992 PL 293602
(43) Veröffentlichungstag der Anmeldung: 01.09.1993
(73) Patentinhaber: Klimek, Rudolf, Prof., PL-31049 Cracow (PL); Klimek, Marek, Dr., PL-31161 Cracow (PL)
(72) Erfinder: Klimek, Rudolf, Prof., PL-31049 Cracow (PL); Klimek, Marek, Dr., PL-31161 Cracow (PL)
(74) Vertreter: Degwert, Hartmut, Dipl.-Phys.

(56) Entgegenhaltungen:
- EP-A- 0 080 417
- GB-A- 2 166 240
- IEEE TRANSACTIONS ON BIOMEDICAL ENGINEERING, Bd.38, Nr.2, Februar 1991, NEW YORK, USA Seiten 199 - 211 AMPARO ALONSO-BETANZOS ET AL, 'Foetos: An Expert System for Fetal Assessment'
- PROCEEDINGS SECOND ANNUAL IEEE SYMPOSIUM ON COMPUTER-BASED MEDICAL SYSTEMS,, 26. Juni 1989, MINNEAPOLIS, USA Seiten 16 - 24 H.R.REY ET AL 'Computer Prediction of Neonatal Outcome and Comparison with Assessments by Physicians and Midwives'

## Beschreibung

Die Erfindung bezieht sich auf ein Gerät zur Bestimmung des optimalen Entbindungstermins eines Fetus mit einer Dateneingabeeinheit für einen wachstumsspezifischen Parameter des Fetus, einer mit der Dateneingabeeinheit verbundenen Bestimmungseinheit, die mit Hilfe des Parameters einen Wert ermittelt, der für den optimalen Entbindungstermin bezeichnend ist, und mit einer Anzeigeeinheit, die den Wert anzeigt.

Die Bestimmung des Entbindungstermins ist eine wichtige Diagnose, die im Verlauf einer Schwangerschaft gestellt wird. Die Messung und Bestimmung eines oder mehreren wachstumsspezifischer Parameter im letzten Schwangerschaftsdrittel läßt einen Rückschluß auf den wahrscheinlichsten Entbindungstermin zu. Der voraussichtliche Entbindungstermin ist z.B. eine Kenngröße für während der Schwangerschaft durchzuführende Therapiemaßnahmen. Er ist auch die Basis um ggf. die Entbindung einzuleiten.

Weit verbreitet wird heute der voraussichtliche Entbindungstermin über eine Ausmessung eines Ultraschallbild des Fetus bestimmt. Dabei wird als wachstumsspezifischer Parameter z.B. der Durchmesser des Kopfes, der Brust- oder Bauchumfang oder auch die Femurlänge gemessen. Aus den Meßwerten wird dann über Tabellen oder Tafeln das Gestationsalter, z.B. die Schwangerschaftswoche, ermittelt, wobei als voraussichtlicher Entbindungstermin die 40. Schwangerschaftswoche zugrundegelegt wird.

In modernen Ultraschallgräten wird aus einem oder mehreren Parameterwerten automatisch der Wert ermittelt und angezeigt, der für den voraussichtlichen Geburtstermin bezeichnend ist. Das kann sowohl die Bezifferung der augenblicklichen Schwangerschaftswoche sein als auch die kalendermäßige Angabe des Entbindungstermins. Der so bestimmte voraussichtliche Entbindungstermin entspricht nach neueren Erkenntnissen, vgl. z.B. einen internen Bericht für die International Federation of Gynaecology (FIGO) von Rudolf Klimek: "Ultrasonography in terms of biological and calendar gestational age" aus dem Jahr 1991, nicht der tatsächlichen Reife der Leibesfrucht. Bei einem langsam wachsenden Fetus kann demnach in der 40. Schwangerschaftswoche die Entwicklung im Mutterleib noch nicht abgeschlossen sein, während es für einen schneller wachsenden Fetus sinnvoll sein kann, auch schon vor der 40. Schwangerschaftswoche eine Geburt einzuleiten.

Der tatsächliche Entbindungstermin kann von dem voraussichtlichen Entbindungstermin erheblich abweichen, wobei die natürliche Bandbreite dieser Abweichung +/- 3 Wochen vom Mittelwert von 40 Wochen beträgt. Dabei muß jedoch betont werden, daß noch 5 % der Geburten außerhalb dieser Grenze liegen. Trotzdem kann in den meisten Fällen auch hier von einem normalen Verlauf der Schwangerschaft gesprochen werden. Diese statistischen Schwankungen der Entbindung von dem mittleren Entbindungstermin sowie die statistischen Unsicherheiten des gemessenen Parameterwertes des Fetus in der Schwangerschaft ergeben eine große Unsicherheit bei der Bestimmung des voraussichtlichen Entbindungstermins.

Der Erfindung liegt nun die Aufgabe zugrunde, ein Gerät anzugeben, mit dem eine genauere Angabe des erwarteten Entbindungstermins sowie die Angabe eines optimalen Entbindungstermins möglich ist. Der optimale Entbindungstermin gibt dann den Zeitpunkt an, an dem ein Fetus seine optimale Reife erreicht hat.

Die Aufgabe wird durch ein Gerät laust Anspruch 1 Weitere vorteilhafte Ausbildungen sind in dem Unteransprüchen angegeben. gelöst.

Dem Gerät liegt die Erkenntnis zugrunde, daß wachstumsspezifische Parameter mit unterschiedlichen Geschwindigkeiten zunehmen und daß daher auch der durchschnittliche Wert des Parameters bei der Geburt in unterschiedlichen Zeitabschnitten erreicht wird. Der durchschnittliche Geburtsparameterwert eines oder mehrerer Parameter gibt nun bei bekannter, d.h. gemessener Wachstumsgeschwindigkeit den optimalen Entbindungstermin an. Dabei entspricht der durchschnittliche Wert des Parameters bei der Geburt dem optimalen Parameterendwert.

Dadurch ist eine wesentlich genauere Aussage über den voraussichtlichen Entbindungstermin möglich, der dann auch den optimalen Geburtstermin bezeichnet.

Bei einer vorteilhaften Ausgestaltung umfaßt die Dateneingabeeinheit eine Umschalteinheit, mit der zwischen der Dateneingabe für den ersten Parameterwert und für den mindestens zweiten Parameterwert umgeschaltet werden kann. Damit können die Parameterwerte frei vorgegeben werden.

Bei einer weiteren vorteilhaften Ausgestaltung umfaßt die Dateneingabeeinheit eine erste und eine zweite Parametereingabeeinheit, über die der erste bzw. zweite Parameter eingebbar ist.

In einer vorteilhaften Ausgestaltung beträgt der zeitliche Abstand mindestens ungefähr eine Woche. Der zeitliche Abstand wird im wesentlichen durch die Genauigkeit bestimmt, mit der der wachstumspezifische Parameter gemessen werden kann und in welchen Bereichen die Zunahmegeschwindigkeit des Parameters liegt.

In einer Ausführungsform umfaßt die Auswerteeinheit einen Tabellenspeicher, in dem für jeweils eine Kombination eines Zunahmegeschwindigkeitswertes und eines Parameterwertes ein Wert gespeichert ist. Damit können auch nicht in mathematische Formeln zu fassende Zusammenhänge zwischen der Zunahmegeschwindigkeit und dem Wert ermittelt werden.

Bei Parametern, deren Verlauf während der Schwangerschaft mathematisch beschreibbar ist, kann in vorteilhafter Weise die Auswerteeinheit eine Rechenschaltung enthalten, die aus jeweils einer Kombination eines Zunahmegeschwindigkeitswertes und eines Parameterwertes über einen vorgegebenen funktionellen Zusammenhang den Wert berechnet. Damit wird der Speicherplatz gering gehalten.

Bei einer besonders vorteilhaften Ausgestaltung ist eine Auswahleinheit mit der Bestimmungseinheit verbunden, wobei mit der Auswahleinheit ein Parameter aus einer Anzahl von verschiedenen Parametern auswählbar ist. Damit kann auf der Grundlage verschiedener Parameter jeweils der optimale Entbindungstermin bestimmt werden, wobei Abweichungen zwischen der unabhängig ermittelten Entbindungsterminen auf Entwicklungsstörungen hinweisen können.

Bei einer weiteren Ausgestaltung wird aus mehreren Parametern ein synthetischer Parameter gebildet, der die Grundlagen zur Bestimmung des optimalen Entbindungstermins bildet. Damit können ggf. statistische Schwankungen der einzelnen Meßwerte ausgeglichen werden.

In einer besonders vorteilhaften Ausgestaltung ist das Gerät Teil eines Ultraschall-Diagnosegeräts, wobei das Ultraschall-Diagnosegerät eine Meßeinheit für den Parameter umfaßt und wobei die Meßeinheit mit der Bestimmungseinheit verbunden ist. Bei der Einbindung des Geräts zur Bestimmung des optimalen Geburtstermin in ein Ultraschallgerät entfällt die manuelle Eingabe, die eine Fehlerquelle sein kann. Außerdem kann der optimale Entbindungstermin schneller bestimmt werden.

In zwei weiteren vorteilhaften Ausgestaltungen bildet die Meßeinheit als Parameter einen Abstand zwischen zwei auf dem Bildschirm einstellbaren Meßmarken oder bildet den Flächeninhalt einer Fläche, deren Umrandung mit Meßmarken festgelegt ist. Damit können aus der Anatomie Parameterwerte des wachstumsspezifischen Parameters bestimmt werden. Der Flächeninhalt einer gegebenen Anatomie gibt einen sehr genauen wachstumsspezifischen Parameterwert.

Bei einer weiteren vorteilhaften Ausgestaltung bildet die Meßeinheit als Parameter einen mit Hilfe einer Dopplermessung ermittelten Strömungswert einer Körperflüssigkeit in einem Gefäßquerschnitt. Damit eröffnet sich die Möglichkeit, zur Bestimmung des optimalen Entbindungstermins auch z.B. die Durchblutung der Plazenta heranzuziehen.

Vier Ausführungsbeispiele der Erfindung werden im folgenden anhand von fünf Figuren erläutert. Es zeigen:
- FIG 1: ein Blockschaltbild eines ersten Ausführungsbeispiels eines Geräts zur Bestimmung des optimalen Entbindungstermins,
- FIG 2: ein Blockschaltbild eines zweiten Ausführungsbeispiels eines Geräts zur Bestimmung des optimalen Entbindungstermins,
- FIG 3: ein Blockschalbild eines dritten Ausführungsbeispiels zur Bestimmung des optimalen Geburtstermins,
- FIG 4: ein in ein Ultraschallgerät integriertes Gerät zur Bestimmung des optimalen Entbindungstermins und
- FIG 5: den funktionellen Zusammenhang zwischen einem wachstumsspezifischen Parameter des Fetus und dem optimalen Entbindungstermin.

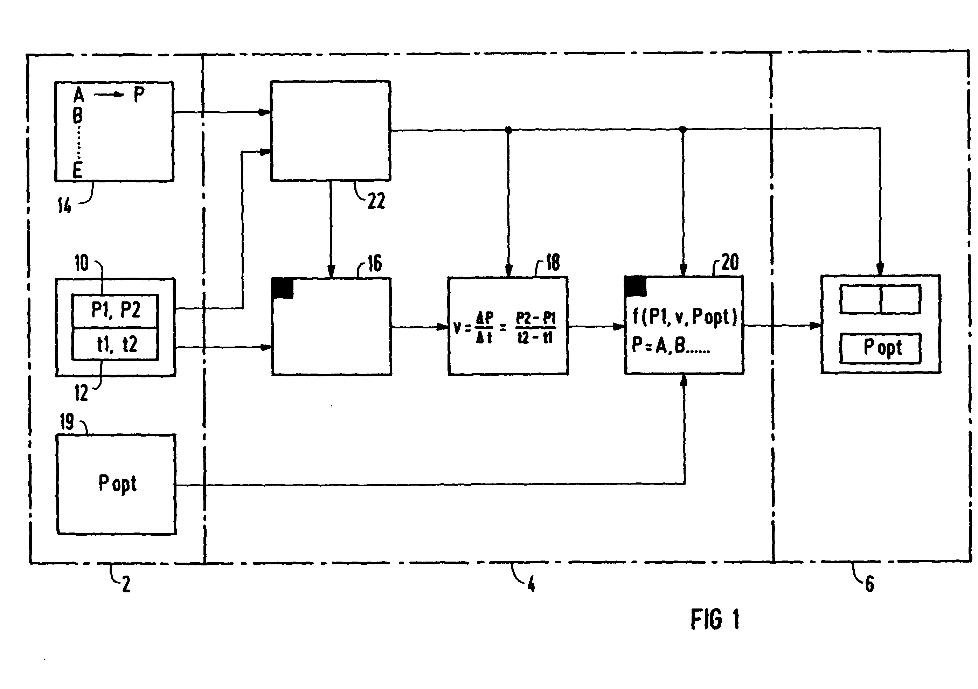

Eine erstes Ausführungsbeispiel des Geräts zur Bestimmung des optimalen Entbindungstermins zeigt FIG 1 im Blockschaltbild. Eine Dateneingabeeinheit 2 dient der Eingabe eines wachstumsspezifischen Parameters des Fetus. Mit der Dateneingabeeinheit 2 ist eine Bestimmungseinheit 4 verbunden, die mit Hilfe des Parameters einen Wert ermittelt, der für den optimalen Entbindungstermin bezeichnend ist. Zur Anzeige des Wertes, der hier die Schwangerschaftswoche beziffert, in der der optimale Entbindungstermin liegt, ist die Bestimmungseinheit 4 mit einer Anzeigeeinheit 6 verbunden.

Die Dateneingabeeinheit 2 umfaßt eine Parameterwert-Eingabeeinheit 10, mit der ein erster Parameterwert P1 und ein in einem zeitlichen Abstand gemessener zweiter Parameterswert P2 des Parameters hingegeben werden kann. Zusätzlich zu der Parameterwert-Eingabeeineheit 10 umfaßt die Dateneingabeeinheit 2 eine Zeiteingabeeinheit 12, über die Meßzeitpunkte tl, t2 eingebbar sind, an denen die Parameterwerte P1, P2 gemessen worden sind. Zur Dateneingabeeinheit 2 gehört auch eine Auswahleinheit 14, mit der ein Parameter aus einer Vielzahl von verschiedenen Parametern A, B ... auswählbar ist. Wachstumsspezifische Parameter sind z.B. der Kopfumfang A, der Brustumfang B, der Bauchumfang C, die Femurlänge D oder der Oxytocinasewert E. Des weiteren ist mit der Auswahleinheit 14 vorgebbar, daß ein aus mehreren Parametern gebildeter synthetischer Parameter zur Bestimmung des optimalen Entbindungstermins herangezogen wird.

Die Bestimmungseinheit 4 umfaßt einen Zwischenspeicher 16, der mit der Parameterwert-Eingabeeinheit 10 und Zeiteingabeeinheit 12 verbunden ist und der die eingegebenen Werte P1, P2, t1, t2 speichert. Eine Recheneinheit 18 ist mit dem Zwischenspeicher 16 verbunden und berechnet aus den Parameterwerten P1, P2 und dem zeitlichen Abstand Δt = t1 - t2 die Zunahmegeschwindigkeit v des Parameters. Da der zweite Parameterwert P2 immer größer als der erste Parameterwert P1 ist, kann die Recheneinheit 18 die eingegebenen Parameterwerte automatisch den entsprechenden Parameterwerten P1, P2 zuordnen. Dasselbe trifft auf die Meßzeitpunkte t1, t2 zu. Aus der Zunahmegeschwindigkeit des Parameters v, aus einem der beiden Parameterwerte P1 oder P2 und einem optimalen Parameterendwert Popt bildet eine mit der Recheneinheit 18 verbundene Auswerteeinheit 20 den Wert, der für den optimalen Entbindungstermin bezeichnend ist. Im Ausführungsbeispiel nach FIG 1 wird die Schwangerschaftswoche beziffert, in der der optimale Entbindungstermin liegt. Dieser Wert kann durch die mit der Auswerteeinheit 20 verbundene Anzeigeeinheit 6 angezeigt werden. Der optimale Parameterendwert Popt ist über Endwerteinstellmittel 19 vorgebbar, da der optimale Endwert z.B. von den Lebensumständen der Schwangeren abhängen kann.

Zur Steuerung des Datenflusses und zur Aktivierung der einzelnen Funktionseinheiten innerhalb des Geräts dient eine Steuerung 22. Als Einangsgrößen sind der Steuerung 22 von der Auswahleinheit 14, der Parameterwert-Eingabeeinheit 10 und der Zeiteingabeeinheit 12 gebildete Steuersignale zugeführt. Daraus werden die Steuersignale für den Zwischenspeicher 16, der Recheneinheit 18, der Auswerteeinheit 20 und die Anzeigeeinheit 6 gebildet.

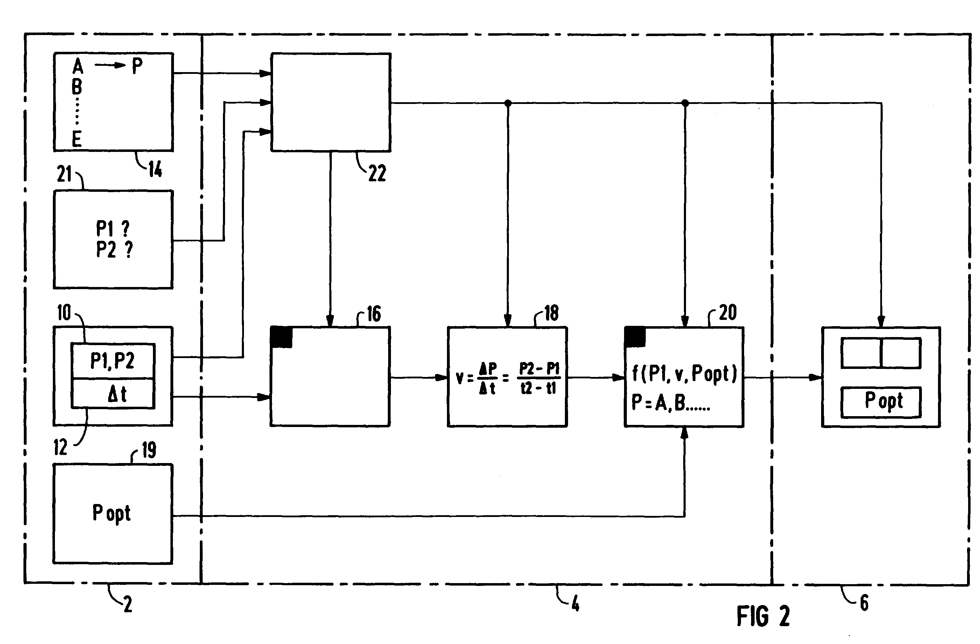

Bei dem in FIG 2 dargestellten zweiten Ausführungsbeispiel ist über eine Umschalteinheit 21 manuell vorgebbar, ob der erste oder zweite Parameterwert P1 bzw. P2 eingegeben wird. Sonst entspricht das zweite Ausführungsbeispiel dem ersten.

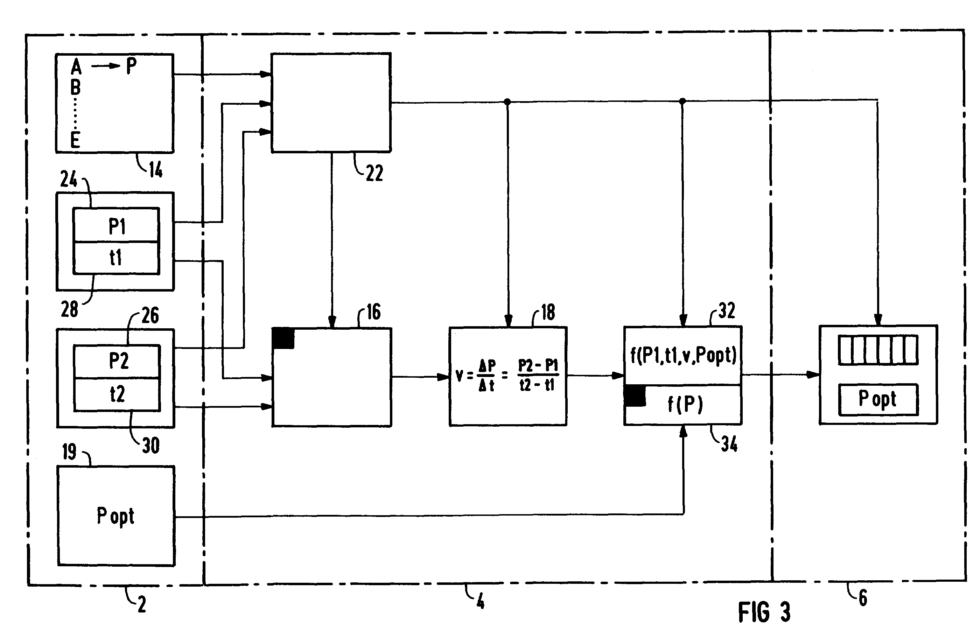

Die in FIG 3 dargestellten Variante umfaßt die Dateneingabeeinheit 2 eine erste Parameterwert-Eingabeeinheit 24 und eine zweite Parameterwert-Eingabeeinheit 26. Die zur Dateneingabeeinheit 2 gehörende Zeiteingabeeinheit ist ebenfalls unterteilt in eine erste Zeiteingabeeinheit 28 und eine zweite Zeiteingabeeinheit 30. Die erste Parameterwert-Eingabeeinheit 24 und die erste Zeiteingabeeinheit 28 sind vorgesehen zur Eingabe eines an einem ersten Meßzeitpunkt t1 gemessenen Parameterwertes P1, und des dazugehörigen Meßzeitpunktes t1. Entsprechend ist die zweite Parameterwert-Eingabeeinheit 26 und die zweite Zeiteingabeeinheit 30 vorgesehen zur Eingabe des im zeitlichen Abstand zum ersten Meßzeitpunkt t1 gemessenen zweiten Parameterwertes P2 und des dazugehörigen Meßzeitpunkts t2. Ebenso wie in den Ausführungsbeispielen nach FIG 1 und 2 werden in dem Ausführungsbeispiel nach FIG 3 die eingegebenen Werte in dem Zwischenspeicher 16 abgelegt. Aus den eingegebenen Werten wird in der Recheneinheit 18 sowohl die Differenz der Parameterwerte P1, P2 als auch die Zeitdifferenz zwischen den Meßzeitpunkten t1,t2 und somit der zeitliche Abstand Δt berechnet. Die daraus bestimmte Zunahmegeschwindigkeit v, einer der Parameterwerte P1 oder P2 und einer der Meßzeitpunkte t1 oder t2 werden einer Rechenschaltung 32 zugeführt, um den optimalen Entbindungstermin kalendermäßig zu berechnen. Der funktionelle Zusammenhang zwischen einem der Parameterwerte P1 oder P2, einen der Meßzeitpunkte t1 oder t2 der Zunahmegeschwindigkeit v des Parameters und dem optimalen Endwert Popt ist für jeden Parameter A, B, ... in einem Festwertspeicher 34 abgelegt.

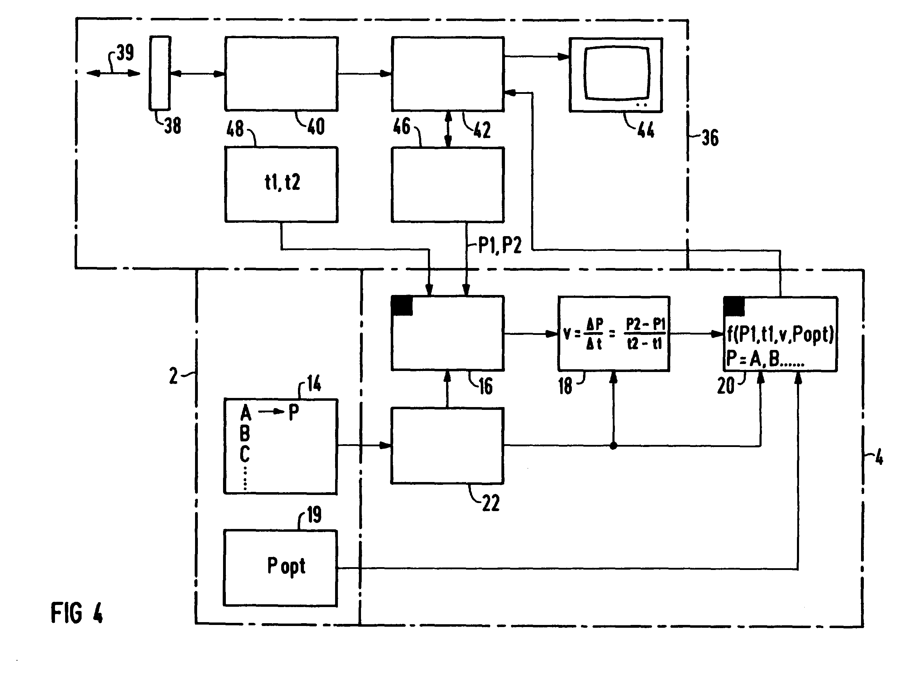

Eine manuelle Dateneingabe entfällt, wenn das Gerät zur Bestimmung des optimalen Entbindungstermins gemäß FIG 4 mit einem herkömmlichen medizinischen Ultraschallgerät gekoppelt oder in ein derartiges Gerät integriert ist. Innerhalb des strichlierten Bereichs 36 ist als Blockschaltbild der Aufbau eines herkömmlichen Ultraschallgerätes angegeben. Das Ultraschallgerät 36 umfaßt einen Ultraschallapplikator 38 zur Aussendung und zum Empfang von Ultraschallsignalen 39, der mit einer Sender-Empfängereinheit 40 verbunden, die die empfangenen Echosignale an eine Bildverarbeitungseinheit 42 weitergibt.

Ein Bildgerät 44 wandelt die von der Bildverarbeitungseinheit 42 gelieferten Bilddaten in sichtbare Bildinformation um. Mit der Bildverarbeitungseinheit 42 ist eine Meßeinheit 46 verbunden, mit der anhand des Schnittbildes die Anatomie vermessen werden kann oder mit der auch Blutströmungsgeschwindigkeiten gemessen werden können. Diese Werte werden als Parameterwerte P1, P2 in den Speicher 16 eingeschrieben und dann weiterverarbeitet wie schon beschrieben wurde. Die Zeitinformation t1, t2 kommt von einem im Ultraschallgerät 36 eingebauten elektronischen Kalender 47. Zusätzlich kann am Ultraschallgerät eine Patientenkennung eingegeben werden, so daß die gemessenen und gespeicherten Werte einem Patienten zugeordnet werden können. Die Weiterverarbeitung erfolgt entsprechend wie anhand von FIG 1 beschrieben ist. Im Unterschied wird jedoch der Wert in Form der Entbindungswoche bzw. des Entbindungsdatums nicht auf einer separaten Anzeigeeinheit, sondern auf einem Bildschirm des Bildgeräts 44 angezeigt. Dazu wird der optimale Parameterendwert Popt und der optimale Entbindungstermin der Bildverarbeitungseinheit 42 zugeführt.

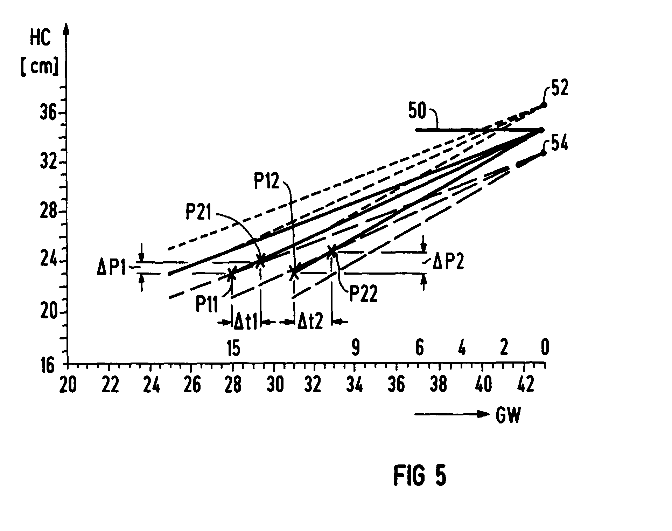

Der funktionelle Zusammenhang zwischen dem Verlauf eines wachstumsspezifischen Parameters während der Schwangerschaft, hier des Kopfumfangs HC, und dem optimalen Entbindungstermins ist im folgenden anhand von FIG 5 beschrieben.

Auf der Abszisse ist die Schwangerschaftswoche GW angegeben, auf der Ordinate der Kopfumfang HC in cm. Der mittlere, d.h. optimale Kopfumfang HC bei der Geburt beträgt 34 cm und ist durch die waagerechte Linie 50 dargestellt. Die statische Schwankungsbreite von +/- 2 cm ist durch die Punkte 52 und 54 dargestellt.

Bei einer ersten Schwangeren wird z.B. in der 28. Schwangerschaftswoche GW der Wert P11 und in der 29. Schwangerschaftswoche GW der Parameterwert P21 gemessen. Der Quotient der Differenzwerte ΔP/Δt gibt die Steigung oder die Wachstumsgeschwindigkeit vl des Kopfumfangs HC an. Empirisch wurde ermittelt, daß der Kopfumfang HC im letzten Drittel in der Schwangerschaft linear wächst. Aus der Wachstumsgeschwindigkeit v, einem der Parameterwerte P11 oder P21 und dem optimalen Parameterendwert Popt kann die Zeitdauer ermittelt werden, in der der mittlere Geburtskopfumfang erreicht wird. Damit kann als optimaler Entbindungstermin die 43. Schwangerschaftswoche angegeben werden.

Bei einer zweiten Schwangeren wurde in der 31. Schwangerschaftswoche der Parameterwert P12 und in der 33. Schwangerschaftswoche der Parameterwert P22 gemessen. Die dazu gehörende Wachstumsgeschwindigkeit v2 = ΔP/Δt ergibt ebenfalls als optimalen Geburtstermin die 43. Schwangerschaftswoche.

Beide Beispiele veranschaulichen ebenfalls einen weiteren Vorteil des Verfahrens, nämlich die Bestimmung des optimalen oder wahrscheinlichsten Entbindungstermins ohne Kenntnis des tatsächlichen Gestationsalters. Es wird nur der wirklich interessierende Zeitraum bis zur Entbindung bestimmt. Am Beispiel der ersten Schwangeren ist zu erkennen, daß vom zweiten Meßzeitpunkt P12 bis zum optimalen Entbindungstermin noch 14 Wochen liegen, während am Beispiel der zweiten Schwangeren vom zweiten Meßzeitpunkt P22 bis zum optimalen Entbindungstermin noch 10 Wochen liegen. Dem liegt die Erkenntnis zugrunde, daß das biologische Gestationsalter, d. h. die verbleibenden Wochen bis zum optimalen Entbindungstermin, als ein Maß für die Reife des Fetus entscheidend ist. Die übrigen, nicht durchgezogenen Linien geben den gesamten Bereich an, in dem der Parameterendwert Popt mit 95 %iger Wahrscheinlichkeit liegt. Die Endpunkte von allen Linien geben das Geburtsdatum an, welches mit 95 %iger Wahrscheinlichkeit zwischen der 37 und 43. Schwangerschaftswoche liegt, wie durch die Linie 50 veranschaulicht.

## Patentansprüche

1. Gerät zur Bestimmung des optimalen Entbindungstermins eines Fetus mit einer Dateneingabeeinheit (2) für einen wachstumsspezifischen Parameter (P;A,B...) des Fetus, einer mit der Dateneingabeeinheit (2) verbundenen Bestimmungseinheit (4), die mit Hilfe des wachstumsspezifischen Parameters (P;A,B...) des Fetus einen Wert ermittelt, der für den voraussichtlichen Entbindungstermin bezeichnend ist, und mit einer Anzeigeeinheit (6), die diesen Wert anzeigt, **dadurch gekennzeichnet**, daß die Dateneingabeeinheit (2) eine Parameterwert-Eingabeeinheit (10) umfaßt, mit der ein erster Parameterwert (P1) eines wachstumsspezifischen Parameters des Fetus und mindestens ein in einem zeitlichen Abstand (Δt) gemessener zweiter Parameterwert (P2) dieses Parameters eingebbar ist, daß die Dateneingabeeinheit (2) eine Zeiteingabeeinheit (12) umfaßt, über die der zeitliche Abstand (Δt) zwischen dem ersten und zweiten Parameterwert eingebbar ist oder über die den zeitlichen Abstand zwischen dem ersten und zweiten Parameterwert bestimmende Zeitpunkte (t1, t2) eingebbar sind, daß die Bestimmungseinheit (4) eine Recheneinheit (18) umfaßt, die aus dem ersten und zweiten Parameterwert (P1,P2) und ihrem zeitlichen Abstand (Δt) die Zunahmegeschwindigkeit (v) des zugehörigen Parameters (P;A,B...) errechnet, und daß die Bestimmungseinheit (4) eine Auswerteeinheit (20) umfaßt, die aus der Zunahmegeschwindigkeit (v) dieses Parameters (P;A,B...), einem dieses Parameterwerte (P1,P2) und einem optimalen Parameterendwert (Popt) einen optimalen Wert bestimmt, der für den voraussichtlichen Entbindungstermin bezeichnend ist, wobei das funktionelle Zusammenhang zwischen einem der Parameterwerte (P1 oder P2), einem der Meßzeitpunkte (t1 oder t2) der Zunahmegeschwindigkeit (v) des Parameters und dem optimalen Parameter endwert (Popt) für jeden Parameter (A, B, ...) in einem Festwertspeicher (34) abgelegt ist.

2. Gerät nach Anspruch 1, **dadurch gekennzeichnet**, daß die Dateneingabeeinheit (2) eine Umschalteinheit (21) umfaßt, mit der zwischen der Dateneingabe für den ersten Parameterwert (P1) und für mindestens den zweiten Parameterwert (P2) umgeschaltet werden kann.

3. Gerät nach Anspruch 1, **dadurch gekennzeichnet**, daß die Dateneingabeeinheit (2) eine erste und eine zweite Parametereingabeeinheit (24 bzw. 26) umfaßt, über die der erste bzw. der zweite Parameterwert (P1,P2) eingebbar ist.

4. Gerät nach einem der Ansprüche 1 bis 3, **dadurch gekennzeichnet,** daß die Dateneingabeeinheit (2) Endwerteinstellmittel (19) zur Einstellung des optimalen Parameterendwertes (Popt) umfaßt.

5. Gerät nach einem der Ansprüche 1 bis 4, **dadurch gekenzeichnet,** daß der optimale Parameterendwert (Popt) auf der Anzeigeeinehit (6) darstellbar ist.

6. Gerät nach einem der Ansprüche 1 bis 5, **dadurch gekennzeichnet,** daß der zeitliche Abstand (Δt) mindestens eine Woche beträgt.

7. Gerät nach einem der Ansprüche 1 bis 6, **dadurch gekennzeichnet,** daß die Auswerteeinheit (20) einen Tabellenspeicher umfaßt, in dem für jeweils eine Kombination eines Zunahmegeschwindigkeitswertes (v), eines Parameterwertes (P1,P2) und des optimalen Parameterendwertes (Popt) ein Wert gespeichert ist.

8. Gerät nach einem der Ansprüche 1 bis 6, **dadurch gekennzeichnet,** daß die Auswerteeinheit (20) eine Rechenschaltung (32) enthält, die aus jeweils einer Kombination eines Zunahmegeschwindigkeitswertes (v) eines Parameterwertes (P1,P2) und des optimalen Parameterendwertes (Popt) über einen vorgebbaren funktionellen Zusammenhang den Wert berechnet.

9. Gerät nach einem der Ansprüche 1 bis 8, **dadurch gekennzeichnet,** daß eine Auswahleinheit (14) mit der Bestimmungseinheit (4) verbunden ist, mit der ein Parameter aus einer Anzahl von verschiedenen Parametern (A, B...) auswählbar ist.

10. Gerät nach Anspruch 9, **dadurch gekennzeichnet**, daß über die Dateneingabeeinheit (2) eine Betriebsart vorgebbar ist, bei der die Bestimmungseinheit (4) aus mehreren ersten und zweiten Parameterwerten einen ersten bzw. zweiten synthetischen Parameterwert bildet und mit einem optimalen synthetischen Parameterendwert den optimalen Wert bestimmt.

11. Gerät nach einem der Ansprüche 1 bis 10, **dadurch gekennzeichnet**, daß das Gerät Teil eines Ultraschall-Diagnosegeräts (36) ist, wobei das Ultraschall-Diagnosegerät (36) eine Meßeinheit (46) für den Parameter (P;A,B...) umfaßt und wobei die Meßeinheit (46) mit der Bestimmungseinheit (4) verbunden ist.

12. Gerät nach Anspruch 11, **dadurch gekennzeichnet**, daß die Anzeigeeinheit durch das Bildgerät (44) des Ultraschallgeräts (36) ersetzt ist.

13. Gerät nach Anspruch 11 oder 12, **dadurch gekennzeichnet**, daß die Meßeinheit (46) als Parameter (P;A,B...) einen Abstand zwischen zwei auf einem Bildgerät (44) einstellbaren Meßmarken nimmt.

14. Gerät nach Anspruch 11 oder 12, **dadurch gekennzeichnet**, daß die Meßeinheit (46) als Parameter (P;A,B...) den Flächeninhalt einer Fläche bildet, deren Umrandung mit Meßmarken festgelegt ist.

15. Gerät nach Anspruch 11 oder 12, **dadurch gekennzeichnet**, daß die Meßeinheit (46) als Parameter (P;A,B...) einen mit Hilfe einer Dopplermessung ermittelten Strömungswert einer Körperflüssigkeit in einem Gefäßquerschnitt nimmt.

## Claims

1. An apparatus for determining the optimal date, of delivery of a foetus with a data input unit (2) for a growth-specific parameter (P; A, B...) of the foetus, a determining unit (4) connected to the data input unit (2), which with the aid of the growth-specific parameter (P; A, B...) of the foetus determines a value which is indicative of the probable date of delivery, and with a display unit (6), which displays this value,
**characterised in that** the data input unit (2) comprises a parameter value input unit (10) with which a first parametervalue (P1) of a growth-specific parameter of the foetus and at least a second parameter value (P2) of this parameter measured at a time interval (Δt) can be entered,
**in that** the data input unit (2) comprises a time input unit (12), via which the time interval (Δt) between the first and second parameter value can be entered or via which instants (t1, t2) determining the time interval between the first and second parameter value can be entered,
**in that** the determining unit (4) comprises an arithmetic-logic unit (18), which from the first and second parameter value (P1, P2) and their time interval (Δt) calculates the rate of increase (v) of the associated parameter (P; A, B...),
**and in that** the determining unit (4) comprises an evaluation unit (20) which from the rate of increase (v) of this parameter (P; A, B...), one of these parameter values (P1, P2), and an optimal final parameter value (Popt) determines an optimal value, which is indicative of the probable date of delivery, with the functional correlation between one of the parameter values (P1 or P2), one of the measuring instants (t1 or t2) of the rate of increase (v) of the parameter and the optimal final parameter value (Popt) being stored in a read-only memory (34) for each parameter (A, B...).

2. An apparatus according to Claim 1,
**characterised in that** the data input unit (2) comprises a change-over unit (21), with which one can switch between the data input for the first parameter value (P1) and for at least the second parameter value (P2).

3. An apparatus according to Claim 1,
**characterised in that** the data input unit (2) comprises a first and a second parameter input unit (24 and 26 respectively), via which the first and respectively the second parameter value (P1, P2) can be entered.

4. An apparatus according to one of Claims 1 to 3,
**characterised in that** the data input unit (2) comprises final value setting means (19) for setting the optimal final parameter value (Popt).

5. An apparatus according to one of Claims 1 to 4,
**characterised in that** the optimal final parameter value (Popt) can be represented on the display unit (6).

6. An apparatus according to one of Claims 1 to 5,
**characterised in that** the time interval (Δt) is at least one week.

7. An apparatus according to one of Claims 1 to 6,
**characterised in that** the evaluation unit (20) comprises a table memory, in which a value is stored for a combination of a rate of increase value (v), a parameter value (P1, P2) and the optimal final parameter value (Popt) in each case.

8. An apparatus according to one of Claims 1 to 6,
**characterised in that** the evaluation unit (20) contains an arithmetic-logic circuit (32), which from in each case a combination of a rate of increase value (v) of a parameter value (P1, P2) and of the optimal final parameter value (Popt) calculates the value via a predeterminable functional correlation.

9. An apparatus according to one of Claims 1 to 8,
**characterised in that** a selection unit (14) is connected to the determining unit (4), with which a parameter can be chosen from a number of different parameters (A, B...).

10. An apparatus according to Claim 9,
**characterised in that** via the data input unit (2) an operating mode can be predetermined, in which the determining unit (4) forms a first and respectively second synthetic parameter value from several first and second parameter values and with an optimal synthetic final parameter value determines the optimal value.

11. An apparatus according to one of Claims 1 to 10,
**characterised in that** the apparatus is a part of an ultrasonic diagnostic instrument (36), with the ultrasonic diagnostic instrument (36) comprising a measuring unit (46) for the parameter (P; A, B...) and with the measuring unit (46) being connected to the determining unit (4).

12. An apparatus according to Claim 11,
**characterised in that** the display unit is replaced by the image device (44) of the ultrasound instrument (36).

13. An apparatus according to Claim 11 or 12,
**characterised in that** the measuring unit (46) takes a distance between two measurement marks which can be set on an image device (44) as parameter (P; A, B...).

14. An apparatus according to Claim 11 or 12,
**characterised in that** the measuring unit (46) forms the area of a surface whose edge is marked by measurement marks as parameter (P; A, B...).

15. An apparatus according to Claim 11 or 12,
**characterised in that** the measuring unit (46) takes a flow value of a body fluid in a vessel cross section determined by means of a Doppler measurement as a parameter (P; A, B...).

## Revendications

1. Appareil pour déterminer la date d'accouchement optimale d'un foetus, équipé d'une unité d'entrée de données (2) pour un paramètre (P;A,B...) spécifique à la croissance du foetus, d'une unité de détermination (4) reliée à l'unité d'entrée de données (2), qui calcule à l'aide du paramètre (P;A,B...) spécifique à la croissance du foetus une valeur qui est significative pour la date d'accouchement probable, et d'une unité d'affichage (6) qui indique cette valeur, caractérisé en ce que l'unité d'entrée de donnés (2) comprend une unité d'entrée de valeurs de paramètres (10), avec laquelle une première valeur de paramètre (P1) d'un paramètre spécifique à la croissance du foetus et au moins une deuxième valeur de paramètre (P2) de ce paramètre, mesurée dans un intervalle de temps (Δt) peuvent être introduites, en ce que l'unité d'entrée de données (2) comprend une unité d'entrée de temps (12), par laquelle l'intervalle de temps (Δt) entre la première valeur de paramètre et la deuxième valeur de paramètre peut être entré ou par laquelle les instants (t1, t2) déterminant l'intervalle de temps entre la première valeur de paramètre et la deuxième valeur de paramètre peuvent être introduits, en ce que l'unité de détermination (4) comprend une unité de calcul (18) qui détermine par le calcul, à partir de la première valeur de paramètre et de la deuxième valeur de paramètre (P1, P2) et de leur intervalle de temps (Δt), la vitesse d'accroissement (v) du paramètre spécifique (P;A,B...), et en ce que l'unité de détermination (4) comprend une unité d'analyse (20) qui, à partir de la vitesse d'accroissement (v) de ce paramètre (P;A,B...), de l'une de ces valeurs de paramètres (P1, P2) et d'une valeur finale optimale de paramètre (Popt), détermine une valeur optimale qui est significative pour la date d'accouchement probable, le rapport fonctionnel entre l'une des valeurs de paramètres (P1 ou P2), entre l'un des instants de mesure (t1 ou t2) de la vitesse d'accroissement (v) du paramètre et la valeur finale optimale du paramètre (Popt) pour chaque paramètre (A, B, ..) est classé dans une mémoire morte (34).

2. Appareil selon la revendication 1, caractérisé en ce que l'unité d'entrée de données (2) comprend une unité de commutation (21) avec laquelle on peut effectuer une commutation entre l'entrée de données pour la première valeur de paramètre (P1) et pour au moins la deuxième valeur de paramètre.

3. Appareil selon la revendication 1, caractérisé en ce que l'unité d'entrée de données (2) comprend une première unité d'entrée de paramètres et une deuxième unité d'entrée de paramètres (24 et 26) par laquelle les première et deuxième valeurs de paramètres (P1,P2) peuvent être entrées, respectivement.

4. Appareil selon l'une quelconque des revendications 1 à 3, caractérisé en ce que l'unité d'entrée de données (2) comprend des moyens d'ajustage de valeur finale (19) pour l'ajustement de la valeur finale optimale de paramètre (Popt).

5. Appareil selon l'une quelconque des revendications 1 à 4, caractérisé en ce que la valeur finale optimale du paramètre (Popt) peut être présentée sur l'unité d'affichage (6).

6. Appareil selon l'une quelconque des revendications 1 à 5, caractérisé en ce que l'intervalle de temps (Δt) est d'au moins une semaine.

7. Appareil selon l'une quelconque des revendications 1 à 6, caractérisé en ce que l'unité d'analyse (20) comprend une mémoire de tableaux dans laquelle une valeur est mémorisée pour respectivement une combinaison d'une valeur de vitesse d'accroissement (v), d'une valeur de paramètre (P1,P2) et de la valeur finale optimale du paramètre (Popt).

8. Appareil selon l'une quelconque des revendications 1 à 6, caractérisé en ce que l'unité d'analyse (20) comporte un circuit de calcul (32) qui calcule la valeur à partir de respectivement une combinaison d'une valeur de vitesse d'accroissement (v), d'une valeur de paramètre (P1,P2) et de la valeur finale optimale du paramètre (Popt) au moyen d'une relation fonctionnelle prédéfinissable.

9. Appareil selon l'une quelconque des revendications 1 à 8, caractérisé en ce qu'une unité de sélection (14) est reliée à l'unité de détermination (4), avec laquelle un paramètre peut être sélectionné à partir d'un nombre de différents paramètres (A, B..).

10. Appareil selon la revendication 9, caractérisé en ce qu'on peut entrer par l'unité d'entrée de données (2) un mode d'exploitation avec lequel l'unité de détermination (4) constitue, à partir de plusieurs premières et deuxièmes valeurs de paramètres, une première et une deuxième valeurs de paramètre synthétiques et détermine la valeur optimale avec une valeur finale de paramètre synthétique optimale.

11. Appareil selon l'une quelconque des revendications 1 à 10, caractérisé en ce que l'appareil est une partie d'un appareil de diagnostic à ultrasons (36), l'appareil de diagnostic à ultrasons (36) comprenant une unité de mesure (46) pour le paramètre (P;A,B...) et l'unité de mesure (46) étant reliée à l'unité de détermination (4).

12. Appareil selon la revendication 11, caractérisé en ce que l'unité d'affichage est remplacée par l'écran (44) de l'appareil à ultrasons (36).

13. Appareil selon la revendication 11 ou 12, caractérisé en ce que l'unité de mesure (46) en tant que paramètre (P;A,B...) occupe un espace entre deux repères de mesure réglables sur un écran (44).

14. Appareil selon la revendication 11 ou 12, caractérisé en ce que l'unité de mesure (46) forme en tant que paramètre (P;A,B...) l'aire d'une surface dont la bordure est définie par des repères de mesure.

15. Appareil selon la revendication 11 ou 12, caractérisé en ce que l'unité de mesure (46) en tant que paramètre (P;A,B...) prend une valeur d'écoulement, calculée à l'aide d'une mesure Doppler, d'un liquide de corps dans une section transversale de récipient.
